**Europäisches Patentamt**

**European Patent Office**

**Office européen des brevets**

(19)

(11) Veröffentlichungsnummer: **0 107 045**
A1

# (12) EUROPÄISCHE PATENTANMELDUNG

(21) Anmeldenummer: **83109408.1**

(22) Anmeldetag: **21.09.83**

(51) Int. Cl.³: **C 07 G 15/00,** C 07 C 103/52, A 23 J 1/20, A 61 K 35/20, A 61 K 37/24

(30) Priorität: **30.09.82 DE 3236264**

(43) Veröffentlichungstag der Anmeldung: **02.05.84** **Patentblatt 84/18**

(84) Benannte Vertragsstaaten: **AT BE CH DE FR GB IT LI LU NL SE**

(71) Anmelder: **Serono Pharmazeutische Präparate GmbH, Merzhauser Strasse 134, D-7800 Freiburg (DE)**

(72) Erfinder: **Voelter, Wolfgang, Prof. Dr., Panoramastrasse 7, D-7400 Tübingen-Hagelloch (DE)** Erfinder: **Lippert, Theodor, Prof. Dr., Erlenweg 38, D-7400 Tübingen (DE)**

(74) Vertreter: **Kraus, Walter, Dr. et al, Patentanwälte Kraus & Weisert Irmgardstrasse 15, D-8000 München 71 (DE)**

(54) **Verfahren zur Herstellung von Relaxin aus Milch.**

(57) Die Erfindung betrifft ein Verfahren zur Gewinnung von Relaxin aus einer wäßrigen, entcaseinierten Milch oder einem Milchkonzentrat. Aus der gekühlten Milch werden die Proteine ausgefällt und abgetrennt und dann wird ein Niederschlag, der das Relaxin enthält, ausgefällt. Dieser relaxinhaltige Niederschlag wird dann in üblicher Weise gereinigt. Die Erfindung betrifft ebenfalls ein Arzneimittel, welches Relaxin enthält.

SERONO PHARMAZEUTISCHE PRÄPARATE GMBH
7800 Freiburg


Verfahren zur Herstellung von Relaxin
aus Milch


Die Erfindung betrifft ein Verfahren zur Herstellung von Relaxin sowie ein Verfahren zur Hochreinigung von Relaxin, das bei dem Verfahren erhaltene Relaxin und ein Arzneimittel, welches das bei dem Verfahren erhaltene Relaxin enthält.

Relaxin wurde erstmals 1926 von Frederick Hisaw als Hormon beschrieben. Er entdeckte den Wirkstoff, als er aus trächtigen Kaninchen gewonnenes Blutserum Meerschweinchen injizierte und eine Relaxation des Symphysenligaments feststellte.

In den folgenden Jahren haben sich kaum irgendwelche Arbeitskreise mit der Darstellung von Relaxin befaßt. Erst mit Beginn der siebziger Jahre gelang es Sherwood und O'Byrne, sehr hoch gereinigtes Relaxin durch säulenchromatographische Methoden zu erhalten. Die Reinigungsverfahren von Sherwood et al. (O.D. Sherwood und E.M. O'Byrne: Arch. Biochem. Biophys. 160, 185 (1974)) sind bis heute die am häufigsten und wirkungsvollsten Methoden zur Gewinnung von hochreinem Relaxin.

Später wurde Relaxin nicht nur in den Organen des Genitaltrakts, sondern auch im Blutserum verschiedener Säugetierespezies (Homo sapiens, Rind, Schwein, Hund, Ratte und Maus) meist während der Schwangerschaft nachgewiesen. Hauptpro-

0107045

duktionsort des Hormons ist wahrscheinlich das corpus luteum des Ovars. Relaxin wird dort zusammen mit Östrogen, Progesteron und Androgen produziert. Für die Isolierung wurden hauptsächlich die Ovarien trächtiger Schweine verwendet, weil hier das Relaxin in beträchtlich höherer Konzentration als bei anderen Säugetieren vorkommt.

Schweine- und Hairelaxin sind bis heute, neben Rattenrelaxin, das einzige Hormon dieser Art, dessen Primärstruktur durch Sequenzierung des isolierten Peptids bekannt ist. Allerdings wurde Relaxin nicht nur bei weiblichen sondern auch bei männlichen Säugetieren (Mensch) wie auch bei männlichen (Huhn) und weiblichen Nichtsäugern (Hai) gefunden.

Bei Mensch und Huhn fand man das Relaxin in den Testes. Bemerkenswert ist, daß Relaxin aber nicht im Hoden oder Blutserum von Bären oder Ratten nachweisbar ist. Relaxin erhöht bei männlichen Spezies die Spermienimmobilität.

Die Konzentration des Relaxins im Blutserum erhöht sich im Laufe der Trächtigkeit bei den Tieren stark. Häufig findet man einen Konzentrationsanstieg kurz vor der Geburt.

Man nimmt an, daß das corpus luteum die häuptsächliche Relaxinquelle von Frauen ist, insbesondere während der Schwangerschaft, wo es im Blut nachweisbar ist. Bei nichtschwangeren Frauen ist die Bildung von Relaxin so gering, daß der Nachweis im Blut kaum möglich ist. Kürzlich wurde das Hormon, von dem man annahm, daß es nur bei Frauen vorkommt, auch in humanem Samenplasma nachgewiesen.

In der folgenden Tabelle ist der Gehalt an relaxinhaltiger Substanz in humaner Milch und im Blutplasma zu unterschiedlichen Zeitpunkten nach der Geburt angegeben.

Tabelle

| Zahl der Patienten | Milch pg/ml (Bereich) | Plasma pg/ml (Bereich) | Zeit nach der Geburt |
|---|---|---|---|
| 9 | 193 (n.n. -700) | 82 (n.n. -203) | Tag 3 |
| 11 | 286 (n.n. -617) | 28 (n.n. -143) | Tag 4 |
| 11 | 391 (n.n. -790) | 19 (n.n. -150) | Tag 5 |
| 7 | 438 (n.n. -810) | 32 (n.n. -223) | Tag 6 |
| 6 | 568 (353 -930) | n.n. | 2-4 Wochen |
| 8 | 464 (177 -741) | n.n. | 5-12 Wochen |
| 4 | 495 (229 -937) | n.n. | 18-34 Wochen |

n.n. = nicht nachweisbar

Es ist bekannt, daß Relaxin vom corpus luteum bei der Schwangerschaft vieler Säugetiere produziert wird. Es war lange Zeit nicht bekannt, ob die Plazenta eine weitere Rohstoffquelle zur Herstellung von Humanrelaxin ist (1. M. X. Zarrow, E. G. Holmstrom und H. A. Salhanick: J. Clin. Endocr. 15, 22 (1955); F. D. Dallenbach und G. Dallenbach-Hellweg: Virchows Arch. Path. Anat. Physiol. 337, 301 (1964) und G. Weiss, E. M. O'Byrne, B.C. Steinetz: Science 194, 948 (1970)). Es scheint nun, daß die menschliche Dezidua Relaxin enthält (M. Bigazzi, F. Nardi, P. Bruni und G. Petrucci: J. Clin. Endocrinol. Metab. 51, 939 (1980); S. Yamamoto, S. C. M. Kwok, F. C. Greenwood und G. D. Bryant-Greenwood: J. Clin. Endocrinol. Metab. 52, 601 (1981); S. Krassnigg, H. K. Rjosk, G. K. Stalla und K. von Werden: Acta Endocrinol. Suppl. 246, 99 (1982)).

Neuere Untersuchungen haben gezeigt, daß dem , insbesondere
dem menschlichen, Relaxin physiologische Wirkungen zukommen
und daß es heute von großem klinischen Interesse ist.

Relaxin zeigt Wirkungen auf den Uterus, beispielsweise beeinflußt es die Uterusmotilität und bewirkt eine Erweichung
der Cervix. In-vitro- und In-vivo-Untersuchungen an Ratten
und Mäuseuteri zeigten eine Verhinderung spontaner Kontraktionen des Myometriums durch Relaxin. Viele biochemische
Veränderungen, die in der Cervix stattfinden, werden durch
Relaxin induziert. Es handelt sich hierbei um die Aufnahme
von Wasser, Glycogen, die Depolymerisation der Grundsubstanz
und die Aktivierung der Kollagenpeptidase.

Relaxin zeigt weiterhin Wirkungen auf das Vaginalephithel,
indem es verhornend wirkt, und Wirkung auf das Wachstum der
Brustdrüsen. Relaxin wirkt mit Progesteron und Östrogen synergistisch und ergibt eine Zunahme des lobuloalveolären Gewebes. Es bewirkt weiterhin eine Erweiterung des Symphysenligaments.

Man unterscheidet Schweine-, Rinder-, Ratten- etc. -relaxine, je nachdem, woraus die betreffende Substanz gewonnen
wurde. Die verschiedenen Relaxine sind alle Polypeptidhormone, die sich teilweise geringfügig in ihrer Aminosäuresequenz, im Molekulargewicht und in ihrer Relaxinaktivität unterscheiden.

Gemeinsam sind dem Relaxin jedoch zwei Peptidketten, welche
durch zwei Cystinreste verknüpft sind. Außerdem zeigen die
Relaxine alle ähnliche pharmakologische Wirkungen.

In der US-PS 2 852 431 wird ein Verfahren zur Extraktion und
Reinigung von Relaxis aus Ovarien trächtiger Tiere beschrieben. In der DE-OS 3 102 487 wird ein Verfahren zur Herstel-

0107045

lung von Humanrelaxin aus Fötusmembranen beschrieben. Diese bekannten Verfahren besitzen jedoch den Nachteil, daß Ausgangsmaterialien verwendet werden, die schwer erhältlich sind und nur in geringem Ausmaß zur Verfügung stehen.Außerdem umfaßt das Verfahren zur Isolierung und Reinigung zahlreiche Stufen und ist somit arbeitsaufwendig und sehr kostspielig. In der Literaturstelle: Hansjürgen Struck "Untersuchungen über Reinigung, Bestimmung und Wirkung des Relaxins" in Forschungsberichte des Landes Nordrheinwestfalen, Nr. 2304 wird über die Reinigung des Relaxins, seine Bestimmung und seine Wirkung berichtet.

Nähere Einzelheiten hinsichtlich der Reinigung und des Nachweises von Relaxin können einer kürzlich erschienenen Zusammenfassung (Gillian D. Bryant-Greenwood, Endocrine Reviews, Bd. 3, Nr. 1, S. 62, 1982) und den dort genannten Literaturstellen entnommen werden. Auf diese Literaturstelle und die dort genannten Zitate wird expressis verbis Bezug genommen.

M. J. Fields et al berichten kürzlich (Annals of the New York Academy of Sciences, Vol. 380, Seite 36 ff (1982)) über ein neues Reinigungsverfahren von Relaxin, bei dem diese Autoren u. a. eine Gelpermeation und Ionenaustauschchromatographie durchführen.

Bis heute gibt es jedoch keine Möglichkeit, Relaxin in größeren Mengen und auf einfache Weise zu gewinnen. Wie oben ausgeführt wurde, stehen die Ausgangsmaterialien zur Isolierung von Relaxin nur in geringen Mengen zur Verfügung und daher ist Relaxin nur in äußerst beschränktem Umfang zugänglich. Die Totalsynthese von Relaxin konnte noch nicht durchgeführt werden, und es ist anzunehmen, daß Relaxin, das durch Totalsynthese hergestellt wird, auch sehr teuer ist. Nachdem dem Relaxin physiologische Wirkungen zu-

0107045

zuschreiben sind, besteht ein Bedarf an einem Verfahren, gemäß dem Relaxin auf einfache Weise erhalten werden kann.

Bei den bekannten Verfahren wird außerdem ein Relaxin erhalten, dessen pharmakologische Wirksamkeit von Herstellungsverfahren zu Herstellungsverfahren schwankt und weiterhin von dem Material abhängt, aus dem das Relaxin gewonnen wird. Dies legt die Vermutung nahe, daß das gemäß den bekannten Verfahren dargestellte und erhaltene Relaxin nicht einheitlich ist und möglicherweise noch Verunreinigungen enthält, die sich in der Vergangenheit nicht haben abtrennen lassen.

Der vorliegenden Erfindung liegt daher die Aufgabe zugrunde, ein Verfahren zur Verfügung zu stellen, gemäß dem Relaxin auf einfache Weise aus billigen, leicht zugänglichen Ausgangsstoffen isoliert und erhalten werden kann.

Erfindungsgemäß soll weiterhin ein Relaxin zur Verfügung gestellt werden, das konstante Eigenschaften aufweist, insbesondere bei den verwendeten Nachweistests immer gleiche Reaktionen zeigt und welches auch gleiche pharmakologische Wirkungen zeigt. Erfindungsgemäß soll ein Relaxin zur Verfügung gestellt werden, dessen Eigenschaften nicht von seinem Herstellungsverfahren und von den zu seiner Herstellung verwendeten Ausgangsmaterialien abhängen. Erfindungsgemäß soll ein Verfahren zur Verfügung gestellt werden, mit dem hochreines Relaxin erhalten werden kann.

Die Erfindung betrifft ein Verfahren zur Gewinnung von Relaxin oder einer relaxinhaltigen Fraktion, das dadurch gekennzeichnet ist, daß man

1) eine wässrige, entcaseinierte Milch, ein Milchkonzentrat oder Milchpulver in aqua dest. auf eine Temperatur im Bereich über der Gefriertemperatur bis zu 15°C kühlt, gegebenenfalls filtriert,

2) zu der gekühlten Flüssigkeit das 0,5- bis 5-fache Volumen an einem in Wasser löslichen, organischen Lösungsmittel zur Ausfällung der Proteine zugibt,

3) den ausgefallenen Niederschlag bei einer Temperatur im Bereich über der Gefriertemperatur bis zu 15°C abtrennt,

4) zu der erhaltenen Flüssigkeit das 1- bis 10-fache Volumen eines auf eine Temperatur von -10°C bis -70°C gekühlten, in Wasser löslichen, organischen Lösungsmittels zugibt, wobei dieses Lösungsmittel das gleiche oder ein anderes, wie bei der Stufe 2) sein kann,

5) das Reaktionsgemisch 0 bis 24 h bei einer Temperatur im Bereich vom Gefrierpunkt des Reaktionsgemisches bis +15°C stehen läßt, und

6) den das Relaxin enthaltenden Niederschlag abtrennt.

Die Erfindung betrifft weiterhin ein Verfahren, das dadurch gekennzeichnet ist, daß man zur Reinigung des Relaxins

7) den bei der Stufe 6) erhaltenen Niederschlag in der 0,5- bis 5-fachen Menge einer wässrigen Pufferlösung mit einem pH-Wert im Bereich von 4,8 bis 6,0 oder in aqua dest. suspendiert, und gegebenenfalls filtriert,

8) gegebenenfalls 0,1 bis 0,5 % Natriumazid, (gegebenenfalls 0,05 bis 5 mM Phenylmethansulfonylfluorid oder -chlorid) zu der erhaltenen Lösung zugibt,

9) die Lösung gegebenenfalls einengt,

10) das Reaktionsgemisch bzw. die das Relaxin enthaltenden Fraktionen über ein Ultramembranfilter filtriert, welches eine Ausschlußgrenze von 10 000 Dalton aufweist und/oder,

11) das Reaktionsgemisch bzw. die das Relaxin enthaltenden Fraktionen einer Ionenaustauschchromatographie unterwirft,

12) das Reaktionsgemisch bzw. die das Relaxin enthaltenden Fraktionen einer Gelfiltration unterwirft,

wobei die Stufen 10, 11 und 12 in beliebiger Reihenfolge und mehrmals durchgeführt werden können und wobei die Stufe 12 zwingend ist und mindestens eine der Stufen 10 oder 11 durchgeführt werden muß und,

13) die das Relaxin enthaltenden Fraktionen einengt und das gereinigte Relaxin gewinnt.

Die Erfindung betrifft weiterhin ein Verfahren zur Hochreinigung von Relaxin, das dadurch gekennzeichnet ist, daß man eine Lösung von gereinigtem Relaxin einer Hochreinigungsstufe unterwirft, indem man

14) Umkehrphasenchromatographie oder/und

15) eine HPLC-Ionenaustauschchromatographie oder/und

16) HPLC-Gelchromatographie oder/und

17) ein Batch-Verfahren mittels Ionenaustauschchromatographie mit anschließender Elution und

18) gegebenenfalls eine Gelchromatographie oder Chromatofokussierung durchführt und,

19) die das Relaxin enthaltende Fraktion einengt und das hochgereinigte Relaxin gewinnt, wobei die Stufen 16 bis 19 gegebenenfalls beliebig wiederholt werden können und wobei mindestens zwei der Stufen 16 und 19 durchgeführt werden müssen.

Die Erfindung betrifft außerdem die bei dem erfindungsgemäßen Verfahren erhaltene relaxinhaltige Fraktion und das bei dem Reinigungs- und Hochreinigungsverfahren erhaltene Relaxin. Sie betrifft außerdem ein Arzneimittel, das dadurch gekennzeichnet ist, daß es neben üblichen Trägerstoffen und Verdünnungsmitteln Relaxin oder eine relaxinhaltige Fraktion, die bei den obigen Verfahren erhalten worden ist, neben üblichen Zusatz- und Trägerstoffen enthält.

Es war überraschend und hat nicht nahegelegen, daß Relaxin in Milchen von Säugetieren insbesondere normaler Kuhmilch vorkommt und daraus auf einfache Weise isoliert werden kann.

Bei dem erfindungsgemäßen Verfahren kann man als Ausgangsmaterialien Säugetiermilch, wie Kuhmilch, Schweinemilch, Stutenmilch, Ziegenmilch oder Schafmilch, verwenden. Erfindungsgemäß ist es bevorzugt, Kuhmilch zu verwenden, da die Kuhmilch in großen Mengen verfügbar ist. Bei dem erfindungsgemäßen Verfahren kann man weiterhin als Ausgangsmaterialien Milchkonzentrate verwenden, das heißt Konzentrate der oben angegebenen Milchen, die beispielsweise erhalten werden, indem man die oben erwähnten Milchen in an sich bekannter Weise, beispielsweise durch Destillation, Lyophilisierung etc., einengt.
Man kann weiterhin als Ausgangsmaterialien Milchlösungen, -suspensionen oder -konzentrate verwenden, die man
erhält, wenn man Milchpulver in Wasser löst oder suspendiert. Milchpulver fällt oft in großen Mengen an und
überraschenderweise wurde gefunden, daß das Relaxin in
dem Milchpulver unverändert enthalten ist.

Bei dem erfindungsgemäßen Verfahren muß
aus der Milch oder dem Milchkonzentrat
erst das Casein entfernt werden. Dies erfolgt nach an
sich bekannten Verfahren. Es war überraschend und hat
nicht nahegelegen, daß das Relaxin nicht zusammen mit
dem Casein, welches ein Protein ist, aus der Milch entfernt wird, sondern daß nach Abtrennung des Caseins das
Relaxin in der Flüssigkeit verbleibt. Man hätte erwarten müssen, daß Relaxin, welches - wie oben aufgeführt wurde - in der Milch ja nur in sehr geringen
Mengen enthalten ist, zusammen mit dem Casein aus der
Milch entfernt würde.

Gemäß dem erfindungsgemäßen Verfahren wird die wäßrige,
entcaseinierte Milch oder das Milchkonzentrat auf eine Temperatur im Bereich über der Gefriertemperatur der Milch
oder des Konzentrats bis zu einer Temperatur von +15°C,
vorzugsweise auf eine Temperatur im Bereich von +1°C bis
+10°C und besonders bevorzugt auf eine Temperatur im Bereich von +2°C bis +6°C, gekühlt. Das Kühlen kann kontinuierlich oder diskontinuierlich erfolgen. Man kann z.B. die
Milch durch einen Schlangenreaktor leiten und dabei kühlen.

Zu der gekühlten Flüssigkeit gibt man dann das 0,5- bis 5-
fache Volumen an einem mit Wasser mischbaren Lösungsmittel
zur Ausfällung der Proteine hinzu. Bevorzugt verwendet man
das 1- bis 3fache Volumen. Die Menge richtet sich danach,
ob Milch oder ein Milchkonzentrat vorliegt, und kann vom

- 11 -

Fachmann leicht bestimmt werden. Die Zugabe des Lösungsmittels kann ebenfalls kontinuierlich oder diskontinuierlich erfolgen. Die ausgefallenen Proteine werden dann in an sich bekannter Weise abgetrennt, wobei die Abtrennung beispielsweise durch Filtrieren, Zentrifugieren, Absetzen etc. erfolgen kann. Zu der erhaltenen Flüssigkeit gibt man dann das 1- bis 10fache Volumen eines auf eine Temperatur von -10°C bis -70°C, vorzugsweise von -10°C bis -65°C, gekühlten, in Wasser löslichen Lösungsmittels. Bevorzugt verwendet man die 2- bis 7fache Volumenmenge. Das mit Wasser mischbare Lösungsmittel kann das gleiche Lösungsmittel sein, wie man es bei der obigen Stufe verwendet. Unter in Wasser löslichen Lösungsmitteln versteht man Lösungsmittel, die mindestens zu 10% in Wasser löslich sind. Beispiele hierfür sind Alkohole, wie Methanol, Ethanol, Isopropanol, n-Propanol, Ketone, wie Aceton, Diethylketon, Methylethylketon und Ether wie Diethylether und Dioxan.

Bei dieser Stufe fällt ein Niederschlag aus, der gleich abgetrennt werden kann. Man kann das Reaktionsgemisch auch noch bei einer Temperatur vom Gefrierpunkt des Reaktionsgemisches bis +15°C stehen lassen, wobei das Stehenlassen während einer Zeit bis zu 20 h erfolgen kann. Dies ist immer dann sinnvoll, wenn die Ausfällung nicht vollständig ist. Es kann sich empfehlen, das Reaktionsgemisch während des Stehenlassens zu rühren. Dies ist jedoch nicht unbedingt erforderlich. Der erhaltene Niederschlag wird in an sich bekannter Weise von der Flüssigkeit abgetrennt, beispielsweise durch Zentrifugieren oder Filtrieren. Der Niederschlag enthält das Relaxin und wird als relaxinhaltige Fraktion bezeichnet. Diese Fraktion kann direkt als Arzneimittel verwendet werden. Da sie aus einem Material stammt, welches als solches als Nahrungsmittel verwendet wird, enthält sie keinerlei Schadstoffe. Überraschenderweise zeigte sich,
daß diese Fraktion ein ausgezeichnetes Antacidum ist und
als Mittel zur Bekämpfung von Magen- und Darmunstimmigkeiten verwendet werden kann.

Aus dem Niederschlag wird dann das Relaxin in reiner
Form gewonnen. Der Niederschlag wird in der 0,5- bis
5fachen, vorzugsweise0,5- bis 2fachen, besonders bevorzugt 1fachen, Menge einer wäßrigen Pufferlösung, die
einen pH-Wert im Bereich von 4,8 bis 5,6 aufweist, suspendiert. Bevorzugt verwendet man einen Ammoniumacetatpuffer mit einem pH-Wert von 5,3. Es können jedoch auch
übliche Phosphatpuffer, die pH-Werte innerhalb der angegebenen Bereiche, vorzugsweise von 4,6 bis 6,2 aufweisen, verwendet werden.

Das erhaltene Reaktionsgemisch wird gegebenenfalls
filtriert und gegebenenfalls mit 0,1 bis 0,5% Natriumazid
und 0,05 bis 5 mM Phenylmethansulfonylfluorid oder
-chlorid versetzt.

Das Relaxin ist jetzt in der Flüssigkeit enthalten. Das
Reaktionsgemisch wird gegebenenfalls über ein Ultramembranfilter, welches eine Ausschlußgrenze von 10 000 bis
100 000 Dalton aufweist, vorzugsweise eine Ausschlußgrenze
von 50 000 besitzt, filtriert. Beispiele für solche
Filter sind die Amiconfilter oder die von der Firma
Berghof gelieferten Filter.

Die erhaltene Flüssigkeit kann an dieser Stelle ebenfalls
gegebenenfalls eingeengt werden. Gemäß den Stufen 10, 11
und 12 wird das Reaktionsgemisch oder eine das Relaxin
enthaltende Fraktion über ein Ultramembranfilter filtriert oder/und einer Ionenaustauschchromatographie und/oder einer Gelfiltration unterworfen. Diese Stufen können beliebig oft durchgeführt werden, wobei es nicht immer erforderlich ist, alle Stufen durchzuführen. Die Gelfiltration ist jedoch zwingend.

Die Chromatographie von relaxinhaltigen Rückständen ist an sich bekannt, und die bekannten Verfahren können verwendet werden. Man kann beispielsweise die Flüssigkeit auf eine Carboxymethylcellulosesäule aufbringen. Die Säule kann vorher mit einem Puffer equilibriert worden sein. Nach der Auftragzeit spült man noch mit Puffer und eluiert dann das Protein mit einem linearen Salzgradienten. Die Reinigung erfolgt, wie in den oben angegebenen Literaturstellen beschrieben. Man erhält jetzt eine Probe, in der das Relaxin in konzentrierter Form zusammen mit anderen Proteinen vorliegt. Diese Probe kann erneut in Pufferlösung, wie oben angegeben, gelöst, chromatographiert und lyophilisiert werden. Die Reinigung erfolgt dann beispielsweise an einer Sephadex-G-Säule, wobei mit Ammoniumacetatpuffer eluiert wird. Man erhält nach mehrmaligen chromatographischen Reinigungen eine relaxinhaltige Probe, die für die verschiedenen Tests verwendet wird und in der das Relaxin in hoher Konzentration vorliegt.

Die Gelfiltration kann auch als Hochdruckgelfiltration durchgeführt werden. Als Gele kann man Sephadex, P-Gele von der Firma Bio Rad oder vergleichbare Gele, TSK-Gele von Merck, etc. verwenden. Bei der Ionenaustauschchromatographie kann man, wie oben ausgeführt, Carboxymethylcellulose oder auch mit Divinylbenzol vernetzte Styrolsulfonsäureharze etc. verwenden.

Gegenstand der Erfindung ist somit, wie oben erwähnt, ebenfalls ein Verfahren zur Hochreinigung von Relaxin, das dadurch gekennzeichnet ist, daß man das Relaxin einer Hochreinigungsstufe unterwirft, indem man eine Umkehrphasenchromatographie, eine HPLC-Ionenaustauschchromatographie, eine HPLC-Gelchromatographie, ein Batch-Verfahren mittels Ionenaustauschchromatographie mit anschließender Elution und gegebenenfalls eine Gelchromatographie durchführt und die das Relaxin enthaltenden Fraktionen einengt. Die oben genannten Stufen für die Hochreinigung können beliebig oft wiederholt und in beliebiger Reihenfolge durchgeführt werden. Es ist auch nicht erforderlich, daß sämtliche Stufen durchgeführt werden.

Bei der Hochdruck- oder Mitteldruckchromatographie verwendet man bevorzugt Ionenaustauschharze oder "reversed-phase" Material ($C_{18}$, $C_6$, $C_2$). Verwendet man bei der Hochdruck- oder Mitteldruckchromatographie Relaxin, welches in der Vergangenheit als reines Relaxin gegolten hat, so erhält man drei Peaks, von denen nur einer pharmakologisch wirksam ist. Verwendet man dagegen bei der Hochdruck- oder Mitteldruckchromatographie Relaxin, welches aus Humanplazenta stammt und aus einer Carboxymethylcellulosesäule abgetrennt wurde, erhält man, wie in der beigefügten Fig. 3 erkennbar ist, vier Peaks, von denen ein Peak pharmakologisch wirksam ist.

Die Hochreinigung des Relaxins erfolgt mit einem präparativen Hochdruck- oder Mitteldruckchromatographiesystem, das speziell für die Ionenaustauschchromatographie von Peptiden und Proteinen an einem Kationenaustauscher ausgelegt ist (W. Voelter, H. Bauer, S. Fuchs und E. Pietrzik: J. Chromatogr. 153, 433-442 (1978); vgl. auch W. Voelter "High Performance Liquid Chromatography in Peptide Research" in der Monographie: High Performance Liquid Chromatography in Protein and Peptide Chemistry, Walter de Gruyter, Berlin, New York 1981). Auf die Offenbarung in dieser Literaturstelle wird expressis verbis Bezug genommen.

Für die Elution des Relaxins wird z.B. ein fünfstufiger Konzentrations- und pH-Gradient von Pyridin-Acetat-Puffer verwendet. Er überstreicht einen Konzentrationsbereich von 0,05 bis 3M Pyridin und einen pH-Bereich von pH 3,0 bis pH6. Bei der "Reversed-phase"-Chromatographie hat sich u.a. ein Acetonitrilgradient bewährt. Zur Detektion werden ca. 4% des Eluats einer kontinuierlichen Partialhydrolyse in N6 Natronlauge unterworfen und entstandene freie Aminogruppen mit einem angesäuerten Ninhydrinreagens bei 570 nm photometrisch detektiert. Dadurch lassen sich auch Peptide ohne freie Aminogruppen und größere Peptide mit höherer Empfindlichkeit nachweisen. Die Nachweisgrenze liegt unter 1 µg je Fraktion.

Durch Lyophilisieren werden die isolierten Fraktionen als salzfreie Produkte direkt gewonnen, da Pyridin-Acetat-Puffer rückstandsfrei verdampfbar sind.

Bei der Trennung des Relaxins an einem stark sauren Ionenaustauscher auf der Basis eines quervernetzten Polystyroldivinylbenzolgerüsts werden mehrere Fraktionen erhalten. Die mittels des Mitteldrucksystems gewonnenen Fraktionen werden im Vakuum eingeengt, in bidest. Wasser aufgenommen und lyophilisiert.

Zum Nachweis des Relaxins werden die bekannten Testverfahren, wie Radioimmunoassay oder Biotest, verwendet. Die Durchführung des Biotests erfolgt gemäß dem Verfahren von Steinitz et al (Endocrinol. 67, 102, 1966), die des RIA gem. E. Loumaye, B. Teuwissen und K. Thomas, Gynecol. Obstet. Invest., 9, 262-267, 1978 .

Untersuchungen haben gezeigt, daß das Relaxin therapeutisch bei verschiedenen pathologischen Zuständen in der Schwangerschaft, wie vorzeitige Niederkunft, drohender Abort und des Verbindungsgewebes, verwendet werden kann. Das Relaxin, insbesondere das Humanrelaxin, ist von äußerster Wichtigkeit für die Herstellung eines homologen Radioimmunoassaybestecks, das die Messung von

0107045

Relaxin in menschlichen Körperflüssigkeiten und Geweben gestattet.

Überraschenderweise wurde jetzt auch gefunden, daß das Relaxin die MAgensaftsekretion hemmt und eine Übersäuerung des Magens vermieden wird. Dies gilt ganz besonders für Relaxin und relaxinhaltige Fraktionen das bzw. die aus Milch, insbesondere aus Kuhmilch, gewonnen werden.

Die folgenden Beispiele erläutern die Erfindung.

B e i s p i e l   1

a) Vorreinigung des Relaxins:

Zu etwa 2 1 frischer gekühlter Rindermilch werden zuerst 600 mg $NaN_3$ und eine Spatelspitze Phenylmethansulfonyl-fluorid gegeben. Danach werden unter Rühren mit einem Magnetrührer langsam innerhalb von 15 Minuten 375 ml konzentrierte rauchende HCl zugesetzt. Die Milch wird mit zunehmender Salzsäurezugabe immer dickflüssiger, und es scheidet sich eine flockige weiße Substanz ab. Man läßt noch weitere 15 Minuten rühren und bringt dann die Flüssigkeit für einen Tag in einen Kühlraum von $+4^{\circ}C$.

Am nächsten Tag wird das ausgefällte Casein durch Filtration oder Abnutschen abgetrennt. Man erhält eine gelbe Flüssigkeit von etwa 2,4 1. Das erhaltene Filtrat wird 3 Stunden lang bei $+4^{\circ}C$ gekühlt. Dazu gibt man 1,8 1 Aceton, welcher ebenfalls 3 Stunden lang bei $+4^{\circ}C$ gekühlt wurde. Diese Lösung läßt man 12 Stunden bei $+4^{\circ}C$ stehen.

In diesem Zeitraum fallen Proteine, welche in diesem Medium unlöslich sind, zum Beispiel $\gamma$-Globuline, aus. Auch diese ausgefallenen Proteine werden abfiltriert. Man erhält dann eine hellgelbe Flüssigkeit von etwa 3,2 1 Volumen. Dieser Lösung werden das 5fache Volumen, also 16 1, Aceton, welches 3 Stunden lang bei $-60^{\circ}C$ gekühlt wurde, zugegeben. Man beobachtet eine sofortige Ausflockung eines weißen Niederschlags. Die Lösung wird etwa 12 Stunden bei $+4^{\circ}C$ stehengelassen. Der Niederschlag wird abfiltriert oder abgenutscht und im Vakuumexikkator über $CaCl_2$ getrock-net. Durch diese Vorgehensweise erhält man etwa 60 g Trockensubstanz, welche das Rohrelaxin darstellt.

b) <u>Reinigung des Relaxins:</u>

Die Trockensubstanz wird in 100 ml eines 50 mMol Ammoniumacetatpuffers mit einem pH-Wert von 5,3, welcher noch 0,02% $NaN_3$ enthielt, aufgelöst. Die überstehende Lösung wird bei 4000 $min^{-1}$ 20 Minuten lang zentrifugiert oder filtriert. Dadurch erhält man etwa 80 ml, die unter Umständen noch filtriert werden müssen, um sie völlig zu klären.

Diese Flüssigkeit bringt man dann mit einer Geschwindigkeit von ca. 25 ml/h auf eine Carboxymethylcellulose-Säule (CM - 52, 2,6 x 40 cm). Die Säule wurde vorher mit 50 mMol $NH_4OOCCH_3$-Puffer vom pH = 5-7, welcher noch 0,02% $NaN_3$ enthält, äquilibriert. Nach der Auftragezeit von etwa 3 Stunden (auch mit Reservoir und Adapter) spült man noch zwei- bis dreimal mit je 1 ml Pufferlösung nach und eluiert dann das Protein mit einem linearen Salzgradienten. Der lineare Salzgradient wird mit einem Gradientenmischer, welcher im Gefäß 1 300 ml 0,1Mol NaCl (oder auch von 0 bis 0,4 M NaCl), gelöst in der obigen Pufferlösung, enthält, und im Gefäß 2 300 ml 0,5 Mol NaCl, gelöst in der obigen Pufferlösung, enthält, hergestellt. Nichtadsorbiertes Protein wird mit 0,1Mol NaCl-Lösung oder reinem Puffer aus der Säule gewaschen, während das Relaxin erst bei einer NaCl-Konzentration von ca. 0,25 bis 0,45Mol/l NaCl von der Säule eluiert wird. Mit einem Fraktionierer wird zeitgesteuert alle 15 Minuten eine neue Fraktion gesammelt. Die relaxinhaltigen Fraktionen, welche photometrisch bei 280 nm detektiert wurden, werden peakweise gesammelt und lyophilisiert.

Das lyophilisierte Protein wird in möglichst wenig Ampuwa ®, welches noch 0,02% $NaN_3$ enthält, gelöst. Man erhält eine gelbe Flüssigkeit von etwa 3 bis 5 ml Volumen. Manchmal enthält diese Lösung noch Rückstände, welche durch Filtration mit einem Weißbandfilter abgetrennt werden müssen. Um die Lösung zu entsalzen, wird sie auf eine Sephadex-G-10-Säule (1,5 x 75 cm) (oder auch P-GDG, oder P-2-säule, 5-6 ml/15 min-Fraktion) aufgetragen. Man chromatographiert mit voller Geschwindigkeit und erhält bei einer Fraktionsdauer von 20 Minuten etwa 10 ml pro Fraktion. Die einzelnen Fraktionen werden wieder bei 280 nm photometrisch durchgemessen, die proteinhaltigen Fraktionen gesammelt und lyophilisiert.

Das aus etwa 10 l Mil-ch gesammelte lyophilisierte und entsalzte Protein wird anschließend in so wenig wie möglich 0,2 Mol Ammoniumacetatpuffer, welcher 0,02% $NaN_3$ enthält, gelöst. Man erhält etwa 5 bis 10 ml einer gelben Flüssigkeit, welche man zur Klärung filtrieren oder zentrifugieren muß. Diese Lösung bringt man auf eine Sephadex-G-50-(super)fine-Säule (100 x 2,6 cm), welche mit 0,2 Mol $NH_4OOCCH_3$/0,02 $NaN_3$ äquilibriert wurde. Die Durchlaufgeschwindigkeit wird auf 4 bis 6 ml pro 20 Minuten eingestellt. Alle 20 Minuten wird mit dem Fraktionierer eine neue Fraktion gesammelt. Die relaxinhaltigen Fraktionen werden bei 280 nm photometrisch detektiert, peakweise gesammelt und lyphilisiert.

B e i s p i e l   2

1000g einer im Handel erhältlichen entcaseinierten Trockenmilch wurden in 10 l Wasser suspendiert. Die Trockenmilchsuspension wurde in einem Kühlschrank bei $+4^{o}$C verschlossen aufbewahrt.

- 20 -

0107045

10 l der Milchsuspension wurden dann, wie in Beispiel 1b) beschrieben, behandelt, und man erhielt eine relaxinhaltige Fraktion in Form eines weißen Pulvers.

Nachweis des Relaxins:

Der Nachweis des Relaxins erfolgte nach dem Radioimmunoassay (RIA) oder nach dem Biotest (vgl. die oben genannten Literaturstellen).

Durchführung des Radioimmunoassays:

Zur Jodierung werden 5µg Relaxin, gelöst in 10 µl 0,1 m Boratpuffer (pH = 8,5) zu 4 mCi Bolton-Hunter-Reagenz gegeben und 15 min. lang bei 0°C geschüttelt. Dazu gibt man 0,5 ml einer 0,2 m Glycinlösung in 0,1 m Boratpuffer und schüttelt wieder bei 0°C 5 min. lang. Die Trennung des Relaxins erfolgt durch Gelchromatographie mit einer Sephadex-G-25-Säule (25 x 1 cm) mit 0,05 m Phosphatpuffer (pH = 7,5). Die das radioaktiv markierte Relaxin enthaltenden Fraktionen werden mit 0,5 ml einer Pufferlösung PM 16 mit 5% Ovalbumin verdünnt und als Stammlösung für den Assay benutzt. Für die Standardkurve werden jeweils 100 µl der verschiedenen Standardlösungen (156 - 20 000 pg Relaxin pro ml PM 16) sowie 300 µl eines SMA-Kontrollserums und 100 µl PM 16 Pufferlösung zugegeben. Dazu gibt man Antikörperverdünnung, die wie folgt gewonnen wird.

Antiserum R6 wird im Verhältnis 1 / 36 000 mit EDTA-Puffer (0,05 m EDTA - $Na_2$ in PM 16-Puffer mit NaOH auf 7,2 eingestellt und mit 1/60 Kaninchenserum versetzt) verdünnt. Man läßt 1 h bei +4°C stehen und fügt dann 100 µl Tracerlösung (Stammlösung des jodierten Relaxins durch Verdünnen mit PM 16/1% Ovalbumin auf ca. 10 000 cpm/100 ml gebracht) zu. Man inkubiert 24 h bei +4°C. Die Fällung des Antigen-Anti-

0107045

körper-Komplexes erfolgt nach der Doppelantikörpermethode mit 200 µl Kaninchenglobulin-Antikörper 1:4 mit PM 16/1% 200 µl Ovalbumin verdünnt. Nach 24 h bei +4°C wird bei 4 000 g zentrifugiert und nach Abdekantieren im Gammezähler gemessen.

Die unspezifische Bindung ist ein Maß für die Wechselwirkung zwischen Antigen und unspezifischen Antikörpern und wird durch Zugabe von 100 µl des EDTA-Puffers zu 300 µl Kontrollserum bestimmt. Man verfährt nach Zugabe von 100µl Tracerlösung, wie oben beschrieben.

Die Nullbindung ist Ausdruck der Bindung zwischen Antigen und Antikörper und wird durch Zugabe von 200 µl PM 16/1% Ovalbumin, 100 µl Antiserumverdünnung und 100% Tracerlösung zu 300 µl Kontrollserum bestimmt.

Ende der Beschreibung.

Patentansprüche

1.    Verfahren zur Gewinnung von Relaxin oder einer relaxinhaltigen Fraktion, dadurch   g e k e n n z e i c h n e t,
daß man

1) eine wässrige, entcaseinierte Milch, ein Milchkonzentrat
   oder eine wässrige Milchpulverlösung auf eine Temperatur
   im Bereich über der Gefriertemperatur bis zu 15°C kühlt,
   gegebenenfalls filtriert,

2) zu der gekühlten Flüssigkeit das 0,5- bis 5-fache Volumen
   an einem in Wasser löslichen, organischen Lösungsmittel
   zur Ausfällung der Proteine zugibt,

3) den ausgefallenen Niederschlag bei einer Temperatur im
   Bereich über der Gefriertemperatur bis zu 15°C abtrennt,

4) zu der erhaltenen Flüssigkeit das 1- bis 10-fache Volumen eines auf eine Temperatur von -10°C bis -70°C gekühlten, in Wasser löslichen, organischen Lösungsmittels zugibt, wobei dieses Lösungsmittel das gleiche oder ein anderes, wie bei der Stufe 2) sein kann,

5) das Reaktionsgemisch 0 bis 24 h bei einer Temperatur im Bereich vom Gefrierpunkt des Reaktionsgemisches bis +15°C stehen läßt, und

6) den das Relaxin enthaltenden Niederschlag abtrennt.

2.    Verfahren nach Anspruch 1, dadurch  g e k e n n -
z e i c h n e t,    daß man zur Reinigung des Relaxins

7) den bei der Stufe 6) erhaltenen Niederschlag in der 0,5- bis 5-fachen Menge einer wässrigen Pufferlösung mit einem pH-Wert im Bereich von 4,8 bis 6,0 oder in aqua dest. suspendiert, und gegebenenfalls filtriert,

8) gegebenenfalls 0,1 bis 0,5 % Natriumazid, gegebenenfalls 0,05 bis 5 mM Phenylmethansulfonylfluorid oder -chlorid zu der erhaltenen Lösung zugibt,

9) die Lösung gegebenenfalls einengt,

10) das Reaktionsgemisch bzw. die das Relaxin enthaltenden Fraktionen über ein Ultramembranfilter filtriert, welches eine Ausschlußgrenze von 10 000 Dalton aufweist und/oder,

11) das Reaktionsgemisch bzw. die das Relaxin enthaltenden Fraktionen einer Ionenaustauschchromatographie unterwirft,

12) das Reaktionsgemisch bzw. die das Relaxin enthaltenden Fraktionen einer Gelfiltration unterwirft,

wobei die Stufen 10, 11 und 12 in beliebiger Reihenfolge und mehrmals durchgeführt werden können und wobei die Stufe 12 zwingend ist und mindestens eine der Stufen 10 oder 11 durchgeführt werden muß und,

13) die das Relaxin enthaltenden Fraktionen einengt und das gereinigte Relaxin gewinnt.

3.    Verfahren nach Anspruch 1, dadurch  g e k e n n -
z e i c h n e t,    daß man zur Hochreinigung von Relaxin eine Lösung von gereinigtem Relaxin einer Hochreinigungsstufe unterwirft, indem man eine

14) Umkehrphasenchromatographie oder/und,

15) eine HPLC-Ionenaustauschchromatographie oder/und,

16) HPLC-Gelchromatographie oder/und,

17) ein Batch-Verfahren mittels Ionenaustauschchromatographie mit anschließender Elution und,

18) gegebenenfalls eine Gelchromatographie durchführt und,

19) die das Relaxin enthaltende Fraktion einengt und das hochgereinigte Relaxin gewinnt, wobei die Stufen 16 bis 19 gegebenenfalls beliebig wiederholt werden können und wobei mindestens zwei der Stufen 16 und 19 durchgeführt werden müssen.

4.   Verfahren nach Anspruch 1,        dadurch  g e -
k e n n z e i c h n e t,    daß man als Milch oder Milchkonzentrat Säugetiermilch oder ein Säugetiermilchkonzentrat oder eine Lösung oder ein Konzentrat eines Säugetiermilchpulvers verwendet.

5.   Verfahren zur Herstellung von Relaxin nach Anspruch 1,
dadurch  g e k e n n z e i c h n e t,    daß man als organisches wasserlösliches Lösungsmittel Ketone,wie Dialkylketone, wie Aceton; Alkohole, Äther,wie Dioxan, Acetonitril,
Dimenthylsulfoxid oder Dimethylformamid oder Gemische dieser Verbindungen verwendet.

6.   Verfahren zur Herstellung von Relaxin nach Anspruch 1,
dadurch  g e k e n n z e i c h n e t,    daß man als Pufferlösung Ammoniumacetatpuffer mit einem pH-Wert von 4,8
bis 5,6, vorzugsweise mit einem pH-Wert von 5,3,oder
Phosphatpuffer mit einem pH-Wert von 4,6 bis 6,2 verwendet.

7.   Verfahren zur Gewinnung von Relaxin, dadurch  g e -
k e n n z e i c h n e t,    daß man

1) eine wässrige, entcaseinierte Milch auf eine Temperatur
   im Bereich über der Gefriertemperatur bis +12°C kühlt,

2) zu der gekühlten Flüssigkeit das 0,5- bis 1,5-fache Volumen Aceton, welches auf eine Temperatur im Bereich
   von -10°C bis +10°C gekühlt ist, zugibt,

3) den ausgefallenen Niederschlag bei einer Temperatur im
   Bereich von der Gefriertemperatur bis +10°C abtrennt,

4) zu der erhaltenen Flüssigkeit das 1- bis 10-fache Volumen Aceton, welches auf eine Temperatur von -10°C bis -70°C gekühlt ist, zugibt,

5) das Reaktionsgemisch 4 bis 20 h bei einer Temperatur im Bereich vom Gefrierpunkt des Reaktionsgemisches bis +10°C stehen läßt,

6) den das Relaxin enthaltenden Niederschlag abtrennt und gewinnt oder

7) den Niederschlag in der 0,8- bis 5-fachen Menge einer wässrigen Pufferlösung mit einem pH-Wert im Bereich von 4,8 bis 6,0 suspendiert.

8) die erhaltene Suspension in einen Niederschlag und eine Lösung auftrennt,

9) die Lösung gegebenenfalls nach Einengen an einer Carboxymethylcellulosesäule unter Verwendung einer Natriumchloridgradientenlösung als Eluierungsmittel eluiert,

10) die das Relaxin enthaltenden Fraktionen vereinigt, gegebenenfalls einengt und lyophilisiert, und

11) gegebenenfalls die Chromatographie und Lyophilisierung wiederholt.

8. Relaxin, dadurch g e k e n n z e i c h n e t , daß es nach einem der Ansprüche 1 bis 7 erhalten worden ist.

0107045

9.   Arzneimittel, dadurch  g e k e n n z e i c h n e t ,
daß es neben üblichen Arzneimittelträgerstoffen und/oder
Hilfsstoffen Relaxin, welches nach einem der Ansprüche 1
bis 7 erhalten worden ist, enthält.

Europäisches
Patentamt

EUROPÄISCHER RECHERCHENBERICHT

0107045

Nummer der Anmeldung

EP    83 10 9408

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| A | Chemical Abstracts Band 80, Nr. 13, 1. April 1974, Columbus, Ohio, USA C.D. SHERWOOD et al. "Purification and characterization of porcine relaxin", Seite 94, Spalte 1, Abstract Nr. 67606x & Arch. Biochem. Biophys., Band 160, Nr. 1, 1974, Seiten 185-196 (Kat. A, D)<br><br>--- | 1,5 | C 07 G   15/00<br>C 07 C  103/52<br>A 23 J    1/20<br>A 61 K   35/20<br>A 61 K   37/24 |
| A | Chemical Abstracts Band 88, Nr. 11, 13. März 1978, Columbus, Ohio, USA M. WEISS et al. "Purification procedure for the peptide hormone relaxin", Seite 224, Spalte 1, Abstract Nr. 71076g & Arch. Gynaekol., Band 223, Nr. 3, 1977, Seiten 2 33-239<br><br>--- | 1 | |
| A | Chemical Abstracts Band 96, Nr. 17, 26. April 1982, Columbus, Ohio, USA M.J. FIELDS et al. "Octadecylsilica and carboxymethyl cellulose isolation of bovine and porcine relaxin", Seite 107, Spalte 2, Abstract Nr. 136066r & Ann. N.Y. Acad. Sci., Band 380, 1982, Seiten 36-46 (Kat. A, D)<br><br>---                    -/- | 1 | |

**RECHERCHIERTE SACHGEBIETE (Int. Cl. ³)**

A 23 J    1/20
A 61 K   35/20
A 61 K   37/24
C 07 C  103/52
C 07 G   15/00

Der vorliegende Recherchenbericht wurde für alle Patentansprüche erstellt.

| Recherchenort | Abschlußdatum der Recherche | Prüfer |
|---|---|---|
| BERLIN | 21-12-1983 | KNAACK M |

Europäisches
Patentamt

**EUROPÄISCHER RECHERCHENBERICHT**

0107045

Nummer der Anmeldung

EP 83 10 9408

## EINSCHLÄGIGE DOKUMENTE

Seite 2

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| A | Chemical Abstracts Band 95, Nr. 3, 20. Juli 1981, Columbus, Ohio, USA T.H. LIPPERT et al. "Immunoreactive relaxin-like substance in milk", Seite 452, Spalte 1, Abstract Nr. 22224q & IRCS Med. Sci.: Libr. Compend., Band 9, Nr. 4, 1 981, Seite 295 | 1 | |
| A | Chemical Abstracts Band 96, Nr. 25, 21. Juni 1982, Columbus, Ohio, USA S. YAMAMOTO et al. "The isolation of relaxin from boar testis", Seite 96, Spalte 2, Abstract Nr. 211088e & Relaxin, Proc. Workshop Chem. Biol. Relaxin, 198 0, Seiten 71-74 | 1,6 | |

RECHERCHIERTE SACHGEBIETE (Int. Cl. ³)

Der vorliegende Recherchenbericht wurde für alle Patentansprüche erstellt.

| Recherchenort | Abschlußdatum der Recherche | Prüfer |
|---|---|---|
| BERLIN | 21-12-1983 | KNAACK M |